Europäisches Patentamt

(19) European Patent Office

Office européen des brevets

(11) Numéro de publication: **0 175 034**
**A1**

(12) **DEMANDE DE BREVET EUROPEEN**

(21) Numéro de dépôt: **84401708.7**

(22) Date de dépôt: **23.08.84**

(51) Int. Cl.⁴: **C 12 P 7/06, C 12 M 1/08**

(43) Date de publication de la demande: **26.03.86**
**Bulletin 86/13**

(84) Etats contractants désignés: **AT BE CH DE GB IT LI NL SE**

(71) Demandeur: **MULTIBIO, Société Anonyme dite, 45, rue de la Chaussée d'Antin, F-75009 Paris (FR)**

(72) Inventeur: **Legesgue, Yves, 34 Grande Rue, 95450 Vigny (FR)**
Inventeur: **Zeana, Alexandru, 10-12, Allée de la Toison d'Or, 94000 Creteil (FR)**

(74) Mandataire: **Cuer, André et al, CABINET CUER 30, rue de Léningrad, F-75008 Paris (FR)**

(54) **Procédé pour la production d'éthanol à partir de substrats sucrés concentrés.**

㊼ Le procédé comprend deux phases successives de fermentation dans deux réacteurs en série, le premier réacteur (1) étant alimenté par un mélange desdits substrats sucrés additionnés de vinasses partiellement épurées en alcool provenant de la phase de distillation, tandis que le second réacteur (3) est alimenté par l'effluent dudit premier réacteur, éventuellement mélangé à une fraction additionnelle de substrats sucrés. La température dans le second réacteur (3) est inférieure à celle du premier réacteur (1).

EP 0 175 034 A1

0175034

## Procédé pour la production d'éthanol à partir de substrats sucrés concentrés.

La présente invention a trait au domaine de le fermentation alcoolique de substrats à haute teneur en hydrates de carbone. Elle concerne plus spécialement la production d'éthanol à partir de mélasses, de jus de cannes concentrés, de jus de betteraves, de produits intermédiaires de sucreries de betteraves ou de produits sucrés concentrés similaires.

On sait quels graves problèmes posent aux distilleries la consommation importante d'énergie et les quantités excessives de vinasses produites. Chaque litre d'alcool à 100 %, produit dans une distillerie de mélasses ou de sirop, entraîne en effet une production de 10 à 12 litres de vinasses.

Les dommages causés à l'environnement par le rejet de ces grandes quantités de vinasses non traitéessont inacceptables. De plus, les distilleries sont obligées de supporter des coûts accrus de traitement de ces effluents.

Le but essentiel de l'invention est de proposer un procédé plus économique pour le traitement de substrats sucrés pour la production d'éthanol consommant moins d'énergie électrique et thermique et produisant beaucoup moins de vinasses que les procédés conventionnels.

Un autre but de l'invention est de fournir un type de fermenteur utilisant les levures fixées sur supports solides en vue d'augmenter la productivité spécifique et d'obtenir un moût alcoolisé ne contenant pas de levure, donc capable de s'affranchir de la phase usuelle de séparation avant distillation.

A cet effet, l'invention a pour objet un procédé pour la production d'éthanol à partir de substrats sucrés concentrés, par fermentation des dits substrats dans un réacteur, conduisant à la production d'un moût et d'un gaz carbonique, et par distillation du dit moût, pour produire de l'éthanol et des vinasses plus ou moins épuisées en alcool, ledit procédé étant caractérisé en ce qu'il comprend deux phases successives de fermentation dans deux réacteurs en série, le premier réacteur étant alimenté par un mélange des dits substrats sucrés additionnés de vinasses partiellement épuisées en alcool provenant de la phase de distillation, tandis que le second réacteur est alimenté par l'effluent dudit premier réacteur, éventuellement mélangé à une fraction additionnelle de substrats sucrés, et en ce que la température dans le second réacteur est inférieure à celle du premier réacteur.

La différence de température entre les deux réacteurs pourra être de l'ordre de 5°C. La température plus élevée du premier réacteur favorise une vitesse élevée de conversion des substrats sucrés en éthanol, tandis que la température plus faible du deuxième réacteur permet d'augmenter la concentration en alcool sans altérer les levures utilisées dans les réacteurs de fermentation.

Les vinasses recyclées au premier réacteur ont une composition apte à maintenir aux valeurs désirées dans ce premier réacteur la concentration en sucres fermentescibles, le rapport produits fermentescibles/produits non fermentescibles et la pression osmotique. Les vinasses utilisées sont prélevées en différents niveaux de la colonne de distillation du moût fermenté, en agissant de manière à faire fonctionner cette colonne dans les conditions les plus économiques.

L'addition éventuelle de substrats sucrés à l'alimentation du second réacteur permet également d'opérer dans ce réacteur dans des conditions optimum de fermentation.

Avantageusement, les levures de fermentation seront fixées sur un support de densité inférieure à celle du moût en fermentation dans les réacteurs et l'alimentation de ces réacteurs s'effectuera de haut en bas, à contre-courant des particules de levure supportée.

0175034

De préférence, les particules de levure supportée seront recyclées à la base des réacteurs de fermentation à l'aide d'un conduit vertical disposé suivant l'axe des réacteurs et équipé d'une vis sans fin entraînée en rotation.

Les levures en excès pourront ainsi se décrocher des supports et s'accumuler par sédimentation à la base des réacteurs, d'où elles pourront être évacuées.

Les dessins annexés illustrent une forme de mise en oeuvre de l'invention. Sur ces dessins :

La figure 1 est un schéma d'ensemble de l'installation utilisée pour cette mise en oeuvre ;

La figure 2 est une vue schématique de détail des réacteurs.

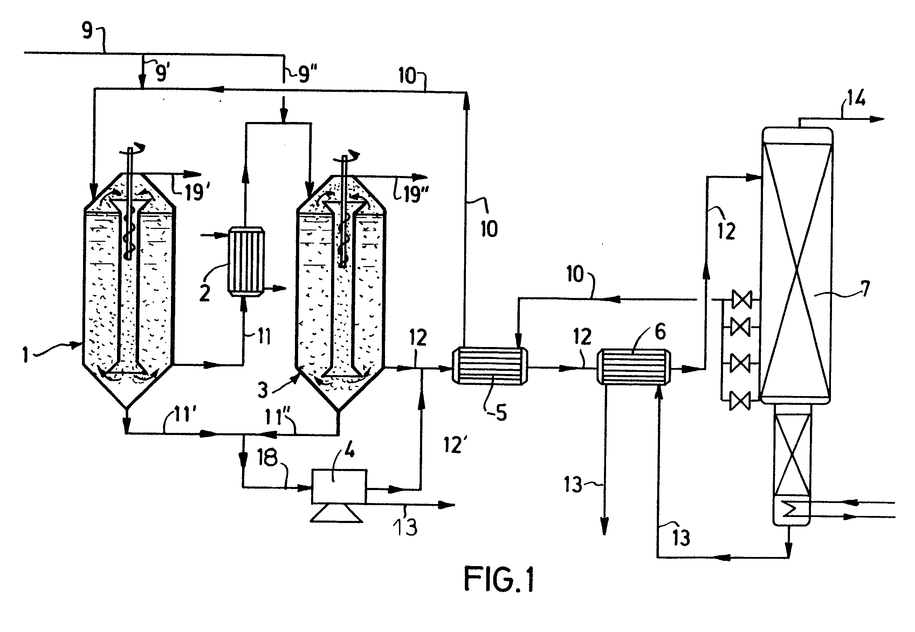

L'installation représentée sur la figure 1 comprend deux réacteurs de fermentation 1 et 3 disposés en série. L'alimentation en substrats sucrés liquides concentrés (mélasses, jus de canne concentré, jus de betterave, produits intermédiaires de sucreries de betteraves) s'effectue à la partie haute des réacteurs 1 et 3, à partir de la ligne 9, par les lignes 9' et 9". Des vinasses recyclées sont amenées par la ligne 10 à la ligne 9' d'alimentation du réacteur 1, tandis que la ligne 9" vient se joindre à la ligne d'évacuation 11 du réacteur 1, disposée à la partie inférieure de ce réacteur, qui alimente le réacteur 3 à sa partie haute. La ligne 11 est refroidie dans un échangeur thermique 2', afin que la température dans le réacteur 3 soit inférieure d'environ 5°C à celle du réacteur 1.

A la partie supérieure des réacteurs 1 et 3 sont prévues des lignes 19' et 19" pour l'évacuation du gaz carbonique produit par fermentation.

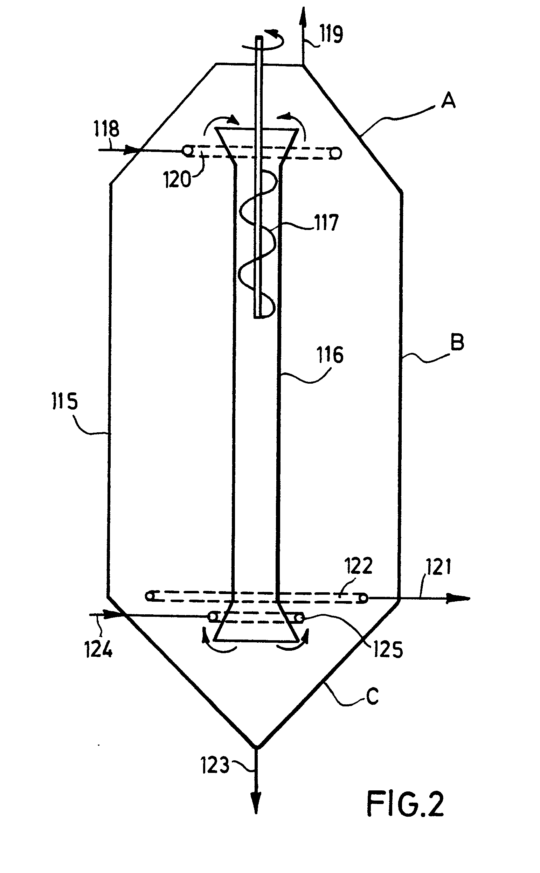

Les réacteurs 1 à 3 contiennent des levures supportées par des particules de masse spécifique inférieure à celle du moût en fermentation, qui ont donc tendance à s'élever dans les réacteurs, à contre-courant du liquide d'alimentation. La structure des réacteurs sera décrite ci-après plus en détail, en référence à la figure 2. On notera simplement que les levures en excès, décrochées de leur support, qui s'accumulent à la base des réacteurs, en sont évacuées par les lignes 11' et 11" et sont dirigées par une ligne 18 vers un système de centrifugation 4, où elles sont séparées du moût restant. Ce moût est acheminé par la ligne 12' vers la ligne 12 de l'effluent du réacteur 3, tandis que les levures sont évacuées du système de centrifugation par une ligne 13.

La ligne 12 évacue le moût fermenté du réacteur 3, à la partie inférieure de celui-ci, vers une colonne de distillation 7, à la partie supérieure de laquelle l'éthanol est évacué par la ligne 14, tandis que les vinasses épuisées en alcool sont évacuées par une ligne 13, à la base de la colonne 7, vers un échangeur thermique 6, qui traverse la ligne 12, en vue de préchauffer le moût fermenté à distiller.

Des vinasses partiellement épuisées en alcool peuvent être prélevées à différents niveaux de la colonne 7, afin d'être recyclées par la ligne 10 à l'alimentation du réacteur 1, en vue d'y maintenir les concentrations des différents constituants du moût aux valeurs désirées. La ligne 10 traverse un échangeur thermique 5 disposé sur la ligne 12 afin de préchauffer l'effluent du réacteur 3.

On notera que la partie basse de la colonne 7 a pour but d'épuiser en alcool les vinasses, avant qu'elles ne soient évacuées par la ligne 13.

Les réacteurs de fermentation sont représentés plus en détail sur la figure 2.

Chaque réacteur 115 est constitué d'une enceinte verticale comprenant trois parties : une partie supérieure conique A, dans laquelle s'effectue la séparation du moût et de la levure sur support et à partir de laquelle le dioxyde de carbone est évacué par la ligne 119 ; une partie cylindrique B, dans laquelle s'effectue la fermentation; et une partie inférieure conique, dans laquelle se déposent par décantation les levures en excès décrochées de leurs supports, en vue de les évacuer par la ligne 123.

0175034

Suivant l'axe du réacteur 115 est disposé un conduit 116 débouchant par l'une de ses extrémités dans la partie A et par l'autre extrémité dans la partie B du réacteur. Une vis sans fin 117 est entraînée en rotation dans le conduit 116, afin de recycler à la base du réacteur les particules supportant les levures.

L'alimentation du réacteur s'effectue à sa partie haute, par un conduit 118 et un anneau 120 percé d'orifices de distribution, coaxial au tube 116, tandis que l'évacuation s'effectue à la partie basse, par un conduit 121 et un anneau collecteur 122, également coaxial au tube 116.

Si nécessaire, une alimentation en air du milieu de fermentation peut être effectuée par une ligne 124 et un anneau de distribution 125, disposé à la base du réacteur coaxialement au tube 116.

La mise en oeuvre de l'invention est illustrée par l'exemple suivant, qui n'a pas de caractère limitatif.

EXEMPLE

On utilise le dispositif décrit ci-dessus pour la fermentation alcoolique de mélasses de canne, dont la composition, en % en poids, est la suivante :

- eaux : 25 %,
- saccharose : 36 %,
- glucose : 7 %,
- fructose : 9%,
- autres matières organiques : 14,5%,
- cendres : 8,5 %.

Le rapport des composés fermentescibles aux composés non fermentescibles est de 2,26%.

On utilise pour la fermentation des souches de levures des genres Saccharomyces et Zymomonas, qui ont une bonne tolérance à l'alcool, notamment Saccharomyces cerevisiae S.T.V.89 et Saccharomyces cerivisiae I.F.O. 0216 ou 0233, sur des supports constitués de copeaux de bois, d'argiles cuites, de copeaux de polychlorure de vinyle, des résines anioniques ou d'autres matériaux présentant une grande aptitude à la fixation des levures, une bonne résistance

0175034

mécanique et une densité inférieure à celle du moût.

La température est d'environ 28°C dans le premier réacteur de fermentation et de 23°C dans le second réacteur, ce qui conduit à des productivités en alcool voisines de 8 kg/h/m$^3$ de réacteur et à des concentrations en alcool du moût fermenté de l'ordre de 80 à 90g/l pour un temps de séjour d'environ 4 à 5 heures. Le taux de recyclage est de 1 à 3 fois le débit de la mélasse.

Cet exemple illustre donc l'efficacité du procédé conforme à l'invention.

- 7 -

REVENDICATIONS

1.- Procédé pour la production d'éthanol à partir de substrats sucrés concentrés, par fermentation des dits substrats dans un réacteur, conduisant à la production d'un moût et d'un gaz carbonique, et par distillation du dit moût, pour produire de l'éthanol et des vinasses plus ou moins épuisées en alcool, ledit procédé étant caractérisé en ce qu'il comprend deux phases successives de fermentation dans deux réacteurs en série, le premier réacteur (1) étant alimenté par un mélange desdits substrats sucrés additionnés de vinasses partiellement épurées en alcool provenant de la phase de distillation, tandis que le second réacteur (3) est alimenté par l'effluent dudit premier réacteur éventuellement mélangé à une fraction additionnelle de substrats sucrés, et en ce que la température dans le second réacteur (3) est inférieure à celle du premier réacteur (1).

2.- Procédé selon la revendication 1, caractérisé en ce que la différence de température entre les deux réacteurs (1, 3) est de l'ordre de 5°C.

3.- Procédé selon l'une des revendications 1 et 2, caractérisé en ce que les levures sont fixées sur un support de densité inférieure à celle du moût en fermentation dans les réacteurs (1, 3) et en ce que l'alimentation des dits réacteurs s'effectue de haut en bas, à contre-courant des particules de levure supportée.

4.- Procédé selon la revendication 3, caractérisé en ce que les particules de levure supportée sont recyclées à la base des réacteurs de fermentation à l'aide d'un conduit vertical (116) disposé suivant l'axe de chacun des réacteurs (115) et équipé d'une vis sans fin (117) entraînée en rotation.

0175034

FIG.1

FIG.2

Office européen
des brevets

**RAPPORT DE RECHERCHE EUROPEENNE**

0175034
Numéro de la demande

EP 84 40 1708

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl 4) |
|---|---|---|---|
| Y | GB-A-2 069 000 (FLETCHER & STEWART) <br> * Figures; page 2, lignes 9-34; page 1, lignes 106-115 * | 1-3 | C 12 P 7/06 <br> C 12 M 1/08 |
| Y | AT-B- 334 857 (VOGELBUSCH GESELLSCHAFT) <br> * Page 3, lignes 1-23; table 1; exemple * | 1 | |
| Y | GB-A-2 104 914 (RESEARCH ASSOCIATION FOR PETROLEUM ALTERNATIVES) <br> * Revendications; figures; exemple 3 * | 1-3 | |
| A | GB-A-2 065 699 (TATE & LYLE LTD.) <br> * Figure 2B; page 7, lignes 5-30 * | 1 | DOMAINES TECHNIQUES RECHERCHES (Int. Cl.4) |
| A | BIOTECHNOLGY AND BIOENGINEERING, volume 13, no. 8, août 1981, pages 1813-1825, New York, US; D. WILLIAMS et al.: "The production of ethanol by immobilized yeast cells" <br> * Page 1817, alinéas 5,6; figure 1; page 1823, alinéa 4 * | 1,3 | C 12 P <br> C 12 M |

-/-

Le présent rapport de recherche a été établi pour toutes les revendications

| Lieu de la recherche <br> LA HAYE | Date d'achèvement de la recherche <br> 31-05-1985 | Examinateur <br> COUCKE A.O.M. |
|---|---|---|

CATEGORIE DES DOCUMENTS CITES

X : particulièrement pertinent à lui seul
Y : particulièrement pertinent en combinaison avec un autre document de la méme catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la méme famille, document correspondant

Office européen des brevets

**RAPPORT DE RECHERCHE EUROPEENNE**

0175034
Numéro de la demande

EP 84 40 1708

Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.⁴) |
|---|---|---|---|
| Y | GB-A-2 091 293 (ZETMEELBEDRIJVEN DE BIJENKORF B.V.) * Figure 1; page 2, lignes 116-130; page 4, lignes 38-56 * | 1 | |
| A | EP-A-0 111 885 (HOECHST A.G.) * Revendications 1,2; page 3, lignes 5-20 * | 3,4 | |
| A | GB-A-2 049 457 (GIZA SpA) * Figures; revendications; page 2, lignes 9-16 * | 4 | |
| E | FR-A-2 550 220 (MULTIBIO S.A.) * En entier * | 1-4 | |

DOMAINES TECHNIQUES RECHERCHES (Int. Cl.4)

Le présent rapport de recherche a été établi pour toutes les revendications

| Lieu de la recherche LA HAYE | Date d'achèvement de la recherche 31-05-1985 | Examinateur COUCKE A.O.M. |
|---|---|---|